# EUROPÄISCHE PATENTSCHRIFT

(11) **EP 2 403 577 B1**
(45) Veröffentlichungstag und Bekanntmachung des Hinweises auf die Patenterteilung: **19.02.2014**
(21) Anmeldenummer: 10718854.2
(22) Anmeldetag: 02.03.2010
(51) Int. Cl.: A61M 16/16

(54) **VERDUNSTUNGSKAMMER MIT ZWISCHENKAMMER SOWIE VERFAHREN**
EVAPORATION CHAMBER WITH INTERMEDIATE CHAMBER, AND METHOD
CHAMBRE D'ÉVAPORATION AVEC CHAMBRE INTERMÉDIAIRE ET PROCÉDÉS CORRESPONDANTS

(30) Priorität: 03.03.2009 DE 102009011137
(43) Veröffentlichungstag der Anmeldung: 11.01.2012
(73) Patentinhaber: Seleon GmbH, 06847 Dessau (DE)
(72) Erfinder: TANTRA, Malinda, 21075 Hamburg (DE); BAECKE, Martin, 06847 Dessau (DE)
(74) Vertreter: Hellmich, Wolfgang
(86) Internationale Anmeldenummer: PCT/DE2010/075020
(87) Internationale Veröffentlichungsnummer: WO 2010/099792

(56) Entgegenhaltungen:
- DE-U1- 29 617 077
- GB-A- 1 294 809
- NL-A- 7 502 668
- US-A- 2 883 511
- US-A1- 2007 240 767

## Beschreibung

Das Gebiet der Erfindung sind Verdunstungskammem, in denen ein niedriger Flüssigkeitspegel erreicht werden kann, Zwischenkammern sowie Verfahren der in den Oberbegriffen der Patentansprüche 1 und 8 genannten Art.

Die Erfindung bezieht sich auf das Gebiet von Verdunstern, insbesondere von Atemluftbefeuchtern für Luftbrillen.

US 2007/0240767 A1 beschreibt einen Autofeed-Mechanismus durch den das Fließen und die Menge einer Flüssigkeit, insbesondere Wasser, in einen Verdunstungsbehälter kontrolliert wird. Dieser Mechanismus besteht aus einem Behälter mit einem Flüssigkeitseinlass, einer Einlasskammer, einer Schwimmerkammer und einem Flüssigkeitsauslass. Ein Ventilsitz, ein Schwimmer und ein Ball erlauben, dass Flüssigkeit von der Einlasskammer in die Schwimmerkammer fließt, wenn der Wasserpegel in der Schwimmerkammer und in dem Verdunstungsbehälter ausreichend gering ist. Der Behälter hat einen Flüssigkeitsauslass, durch den die Flüssigkeit in den Verdunstungsbehälter fließt. Der Boden der Schwimmerkammer weißt keinen Flüssigkeitsauslass auf.

Die Schrift US 2,883,511 beschreibt einen Luftbefeuchter, der aus einer Heizkammer und einer Schwimmerkammer besteht. Die Heizkammer hat eine einteilige zylindrische Außenwand und eine konzentrische Innenwand. Extern an der Heizkammer sind Befestigungsteile angebracht, an denen wiederum die Schwimmerkammer befestigt ist. Eine Öffnung in einer Wand der Schwimmerkammer weist einen Flüssigkeitsanschluss auf. Der Wasserfluss im Flüssigkeitsanschluss wird durch ein gebräuchliches Schwimmerventil geregelt. Die Öffnung in dem Boden der Schwimmerkammer ist mit einem Rohr mit der Heizkammer verbunden.

Ein Beispiel für den Einsatz des Autofeed-Mechanismus in einer Befeuchterkammer eines Beatmungsgerätes ist die Befeuchterkammer MR 290 von Fisher & Paykel mit einem Schwimmkörper. Diese Befeuchterkammer wird zwischen dem Beatmungsgerät und dem Patienten angeschlossen. Sie besteht aus vier wichtigen Teilen: einem Schwimmkörper, einem Gaseintritt- sowie Gasaustritt-Durchlass und einer Leitung für die Wasserzufuhr aus einem Sterilwasserbehälter. Das trockene Atemgas wird von dem Beatmungsgerät in die Befeuchterkammer geleitet, bevor das befeuchtete Atemgas an einen Patienten abgegeben wird. Das Atemgas strömt über das von einer Heizplatte erwärmte Wasser in der Befeuchterkammer. Die erforderliche Atemgasfeuchtigkeit kann über die Wassertemperatur eingestellt werden, die manuell geändert werden kann. Eine Änderung der Wassertemperatur verursacht auch eine Veränderung der Temperatur des Atemgases. Mittels eines durch den Schwimmkörper betätigten Nadelventils wird die Öffnung der Leitung für die Wasserzufuhr bei Erreichen eines Sollwasserfüllstands verschlossen. Während des Betriebs nimmt das im Vorratsbehälter befindliche Wasser aufgrund der über den Atemgasstrom abtransportierten Wassermoleküle ab. Der Schwimmer sinkt mit der Abnahme des Wasserspiegels, so dass das Nadelventil die Leitung öffnet und Wasser über die Leitung in den Vorratsbehälter nachfließen kann. So wird in der Befeuchterkammer der Wasserfüllstand nahezu konstant gehalten. Nachteilig ist, dass die relativ großen Schwimmer einen beträchtlichen Teil der Wasseroberfläche bedecken und zudem eine gewisse Eintauchtiefe benötigen.

Es ist Aufgabe der Erfindung eine Zwischenkammer zu schaffen, in der ein niedriger Flüssigkeitspegel erreicht werden kann.

Diese Aufgabe wird durch die Lehre des unabhängigen Anspruchs 1 gelöst.

Bevorzugte Ausführungsformen der Erfindung sind Gegenstand der Unteransprüche.

Vorteilhaft an einer Verdunstungskammer mit einer Zwischenkammer, die ein Pegelventil aufweist, das im Betrieb aus einem Flüssigkeitsanschluss Flüssigkeit in die Zwischenkammer nachlaufen lässt, so dass der Flüssigkeitspegel in der Zwischenkammer zwischen einem Minimalpegel und einem Maximalpegel liegt, ist, dass die Verdunstungskammer auch aus Sterilwasserbehältern versorgt werden kann, die sogar deutlich höher als die Verdunstungskammer angebracht werden können. Darüber hinaus deckt der Schwimmer die Wasseroberfläche nicht teilweise ab, über die die Luftbefeuchtung erfolgt. So wird eine wesentliche größere Wasserfläche für den Kontakt zur Luft bereit gestellt, ohne dabei die Grundfläche des Wasserbehälters zu vergrößern. Damit kann eine höhere Befeuchtungsleistung erzielt werden. Weiterhin kann der Wasserfilm dünner gehalten werden, so dass geringere Aufheizzeiten möglich sind und auch die Regelung des Systems beschleunigt werden kann.

Wird zwischen Verdunstungskammer und Sterilwasserbehälter eine Ausgleichsleitung vorgesehen, die an einem Ausgleichsanschluss an der Verdunstungskammer angeschlossen werden kann, kann die Verdunstungskammer auch bei erheblichen Überdruck gegenüber dem Umgebungsdruck betrieben werden. Dies ist beispielsweise für TNI® nötig.

Ein Pegelventil kann beispielsweise einfach und kostengünstig durch ein von einem Schwimmer betätigtes Ventil realisiert werden.

Vorteilhaft an der Befeuchtung von Gas im Raum zwischen einer Wanne und dem Boden einer Zwischenkammer ist, dass die Oberfläche eines in der Wanne stehenden Wasserfilms nicht durch einen Schwimmer reduziert wird. Außerdem kann der Wasserfilm in der Wanne dünner sein als der Sollwasserpegel in der Zwischenkammer hoch ist, weil der Wasserfilm keinen Auftrieb für einen Schwimmer erzeugen muss.

Durch die Verwendung eines Ausgleichsrohrs zusätzlich zu einem Flüssigkeitsauslass wird das Nachlaufen von Wasser und der Druckausgleich örtlich getrennt, so dass die Dicke des Wasserfilms in der Wanne exakter eingehalten wird.

Durch das Führen des Schwimmers durch das Ausgleichsrohr kann das Ausgleichsrohr in vorteilhafter Weise eine zusätzliche Funktionen übernehmen. Hierdurch wird der Zusammenbau und die Desinfektion der Zwischenkammer vereinfacht, weil die Zwischenkammer keine zusätzlichen Führungen aufweisen muss, damit insgesamt weniger Kanten und eine kleinere Oberfläche aufweist. Zusätzlich wird der Schwimmer nicht durch aufsteigende Luftblasen gestört.

Dadurch, dass die Oberfläche des Schwimmers nach außen hin abfällt, wird in überraschend einfacher Weise vermieden, dass sich Wasser auf dem Schwimmer sammelt.

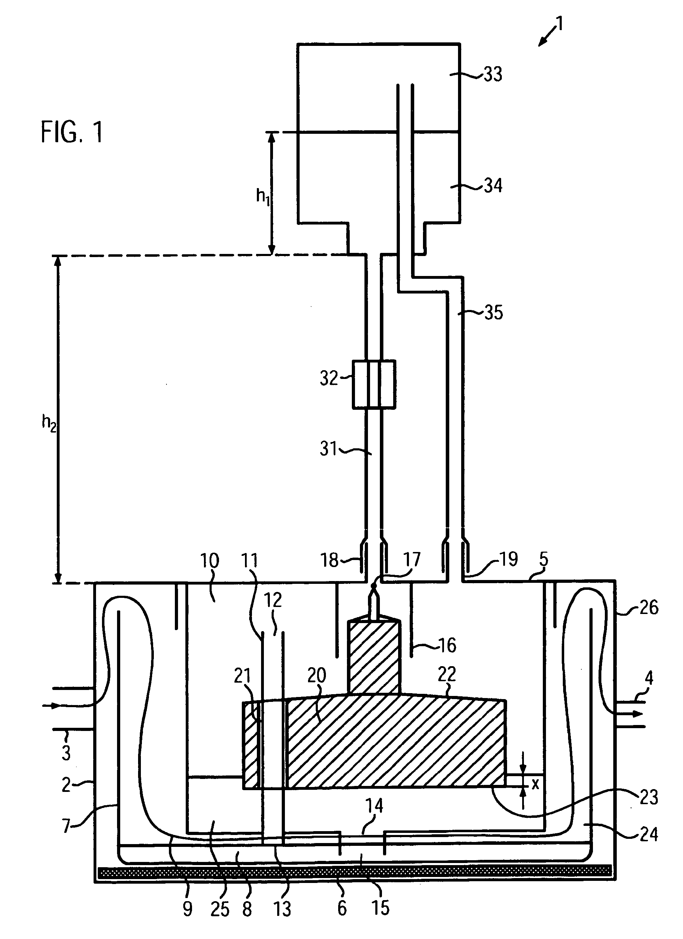

Im Folgenden werden bevorzugte Ausführungsformen der Erfindung unter Bezugnahme auf die beiliegende Zeichnung näher erläutert. Dabei zeigt
Fig. 1 einen erfindungsgemäßen Atemgasbefeuchter.

Der in der Figur 1 gezeigte erfindungsgemäßen Atemgasbefeuchter 1 besteht im Wesentlichen aus einer Verdunstungskammer 2 sowie aus einem Sterilwasserbehälter 33, der über eine Wasserleitung 31 und eine Ausgleichsleitung 35 an einem Wasseranschluss 18 beziehungsweise einem Ausgleichsanschluss 19 der Verdunstungskammer 2 angeschlossen ist. Die Wasserleitung 31 besteht typischerweise aus einem flexiblen, transparenten Kunststoffschlauch und kann durch eine Rollklemme 32 verschlossen werden.

Die Verdunstungskammer 2 umfasst ein Gehäuse 26, einen Deckel 5, eine Heizplatte 6, eine Wanne 7 sowie eine Zwischenkammer 10. Das Gehäuse 26 bildet in Figur 1 links einen Gaseinlass 3 und rechts einen Gasauslass 4. Die Luftströmung 9 vom Gaseinlass 3 zum Gasauslass 4 über einen Wasserfilm 8 ist ebenfalls eingezeichnet. Die Zwischenkammer 10 weist an ihrem Boden einen Wasserauslass 14 mit einem unteren Ende 15 auf. Ferner ist am Boden der Zwischenkammer 10 ein Ausgleichsrohr 11 senkrecht zum Boden, also vertikal angebracht, so dass im Betrieb Atemgas durch das Ausgleichsrohr 11 in das Innere der Zwischenkammer 10 eintreten kann. Das Ausgleichsrohr 11 weist ein oberes Ende 12 und ein unteres Ende 13 auf. Der Wasseranschluss 18 sowie der Ausgleichsanschluss 19 führen in das Innere der Zwischenkammer 10. Der Wasseranschluss 18 kann durch einen Nadelventil 17 verschlossen oder geöffnet werden. Das Nadelventil 17 wird von einem Schwimmer 20 betätigt. Der Schwimmer 20 wird oben durch eine Führung 16 sowie links durch das Ausgleichsrohr 11 geführt, wobei das Ausgleichsrohr 11 durch eine Aussparung 21 im Schwimmer 20 verläuft. Die Oberseite 22 des Schwimmers 20 fällt vom Zentrum des Schwimmers 20, das sich unterhalb des Nadelventils 17 befindet, nach außen hin mit einem Winkel von 10° ab, damit sich auf der Oberseite des Schwimmers keine Wassertropfen oder gar Wasserpfützen ansammeln. Die Zwischenkammer kann aus hartem Kunststoff hergestellt werden.

Im Betrieb fließt aus dem Sterilwasserbehälter 33 Wasser durch die Wasserleitung 31 Wasser in die Zwischenkammer 10 und wird hier mit dem Bezugszeichen 25 bezeichnet. Ist der Wasserpegel in der Zwischenkammer 10 ausreichend hoch, erzeugt der Schwimmer 20 ausreichend Auftrieb, um das Nadelventil 17 zu schließen. So wird in der Zwischenkammer 10 für einen ungefähr konstanten Wasserpegel gesorgt.

Ein Teil des Wassers 25 fließt durch den Wasserauslass 14 in die Wanne 7 und bildet dort den Wasserfilm 8. Der Raum oberhalb des Wasserfilms 8 in der Wanne 7 wird als Verdunstungsraum 24 bezeichnet. Das durch das Einfließen von Wasser in die Wanne 7 verdrängte Atemgas strömt durch das Ausgleichsrohr 11 in die Zwischenkammer 10, bis der Wasserfilm 8 so dick ist, dass er das untere Ende 13 des Ausgleichsrohrs 11 verschließt. In der Folge fließt erst wieder Wasser von der Zwischenkammer 11 in die Wanne 7 nach, wenn der Wasserfilm 8 durch Verdunstung dünner geworden ist und er das untere Ende 13 des Ausgleichsrohrs 11 wieder freigibt. Wie oben erwähnt, kann man diese Dickenregelung des Wasserfilms 8 auch als Vogeltränkenprinzip bezeichnen.

Für die Dicke des Wasserfilms 8 liegt ein Sollwert bei etwa 7mm, ein Minimalwert bei 5 mm und ein Maximalwert bei 15 mm. Dieser Wert ist abhängig von der Ausdehnung des Wasserfilms 8, also den Abmessungen der Wanne 7. Bei einer Neigung des Geräts von 15° sollte die Neigung des Geräts die Grenzfläche zwischen Luft und Wasserfilms möglichst nicht reduzieren. Es sollte also noch immer der gesamte Boden der Wanne 7 mit Wasser bedeckt sein und der Wasserfilm die Unterseite der Zwischenkammer noch nicht berühren. Deshalb soll die Dicke des Wasserfilms maximal halb so groß wie der Abstand zwischen der Unterseite der Zwischenkammer 10 und der Oberseite des Bodens der Wanne 7 sein. Bei einer Dicke des Wasserfilms von 7 mm muss also das untere Ende 13 des Ausgleichsrohrs 11 mindestens 7 mm aus dem Boden der Zwischenkammer 10 nach unten herausragen. Der Wasserauslass 14 muss etwas länger, also beispielsweise 10 mm sein.

Der Wasserfilm 8 und die Wanne 7 wird durch eine am Boden des Gehäuses 26 befindliche Heizplatte 6 beheizt. Über die Temperatur des Wasserfilms 8 und damit über die Temperaturen der Heizplatte 6 oder die der Heizplatte 6 zugeführte Heizleistung lässt sich in bekannter Weise die Feuchtigkeit des Atemgases steuern.

Die im folgenden erwähnten Drücke sind als Überdruck gegenüber dem Umgebungsluftdruck zu verstehen. Für TNI® sind 0 bis 100mbar Druck erforderlich, um in den dünnen Schläuchen einer Luftbrille einen ausreichenden Luftfluss zu erzeugen. Diese bis zu 100mBar herrschen im Verdunstungsraum 24. Deshalb muss der Deckel 5 gegenüber dem Gehäuse 26, der Anschluss der Wasserleitung 31 am Wasseranschluss 18 und am Sterilwasserbehälter 33 sowie der Anschluss der Ausgleichsleitung 35 am Ausgleichsanschluss 19 und am Sterilwasserbehälter 33 druckdicht ausgelegt sein. Bezüglich der Druckdichtigkeit sind drei Bereiche zu unterscheiden:
1. der von Luft durchströmte Bereich, d.h. im Wesentlichen die Verdunstungskammer 2:
   Deren Abdichtung gegen den Deckel 5 und andere Bauteile darf eine Undichtigkeit von kleiner 5% des Soll- Durchsatzes an Luft betragen.
2. der wasserführende Bereich, d.h. Sterilwasserbehälter 33 mit Wasserleitung 31 Ausgleichsleitung 35 und Zwischenkammer 10:
   Die Abdichtung dieser Bauteile gegeneinander darf nur Undichtigkeiten von jeweils maximal 5ml Wasser/24h bei 150mbar Überdruck gegenüber dem Umgebungsdruck oder 50ml Luft/24h bei 100mbar Überdruck gegenüber dem Umgebungsdruck aufweisen.
3. das Nadelventil 17:
   Das Nadelventil 17 sollte eine Undichtigkeit nicht größer als 50ml Wasser/24h bei 10mbar durch die anstehende Wassersäule aufweisen.

Die Dichtigkeitsanforderungen dienen dazu, die Durchnässung des umgebenden Bereiches und die Flutung des Atemgasweges mit Wasser zu vermeiden.

Als Sterilwasserbehälter 33 können sowohl Flaschen als auch Beutel verwendet werden. Beutel werden über die Ausgleichsleitung 35 aufgeblasenen. Falls die Höhe h₃, also der Höhenunterschied zwischen der Oberfläche des Wasserfilms 8 und dem Wasserpegel in der Zwischenkammer 10, 1,5 cm beträgt, ist der Druck in der Zwischenkammer 10 über dem Wasser 25 um 1,5mbar kleiner als der Druck im Verdunstungsraum 24, was meist vernachlässigt werden kann. Der Druck in der Zwischenkammer 10 über dem Wasser 25 wird über die Ausgleichsleitung 35 in den Sterilwasserbehälter 33 übertragen. Der Druck, den das Nadelventil 17 verschließen muss, ergibt sich aus der Höhe der Wassersäule über dem Nadelventil 17, die in Figur 1 (h₂+h₁) beträgt. Oft ist der Sterilwasserbehälter 33 1,5m über dem Nadelventil 17 angeordnet und der Füllstand im Sterilwasserbehälter 33 schwankt zwischen 0 und 20 cm, so dass das Nadelventil einen Druck von 150 bis 170mBar verschließen muss. Hierdurch ergibt sich für die zum Schließen erforderliche Kraft, damit für den auf den Schwimmkörper wirkenden Auftrieb und damit für den Wasserpegel in der Zwischenkammer 10 je ein gewisser Bereich. Der jeweilige Wert hängt insbesondere vom Füllstand im Sterilwasserbehälter, also von h₁, aber auch h₂ ab. Diese Bereiche werden noch größer, wenn man berücksichtigt, dass es ja im Nadelventil 17, zwischen dem Schwimmer 20 und der Führung 16 sowie zwischen der Aussparung 21 und dem Ausgleichsrohr 11 Reibung gibt. In jedem Fall ergibt sich für den Wasserpegel in der Zwischenkammer 10 einen Maximalpegel und einen Minimalpegel. Zwischen dem Maximalpegel und dem Minimalpegel kann man einen beliebigen Sollpegel festlegen, der beispielsweise das arithmetische Mittel aus Maximalpegel und Minimalpegel sein kann.

Mittels einer Rollklemme 32 kann die Wasserleitung 31 verschlossen werden, so dass bei geöffnetem Deckel 5 kein Wasser 34 aus der Wasserleitung 31 austritt.

In einer anderen Ausführungsform ist das Ausgleichsrohr 11 nicht vorhanden. In dieser Ausführungsform gelangen Atemgasblasen durch den Wasserauslass 14 in die Zwischenkammer 10. In dieser Ausführungsform wird vorteilhafter Weise auch die Unterseite 23 des Schwimmers vom Zentrum des Schwimmers 20 nach außen hin beispielsweise um 10° ansteigen, damit sich unter dem Schwimmer keine Atemgasblasen ansammeln.

Die maximalen Maße des Schwimmers einer Ausführungsform betragen in mm: Breite: 56, Länge: 94, Höhe: 34; daraus ergibt sich eine maximales Schwimmervolumen von 236880 mm³.

Nachfolgend ist die benötigte Eintauchtiefe x unter Benutzung der maximalen Breite und Länge des Schwimmers je nach Schwimmergewicht m tabellarisch dargestellt. Ein Sicherheitsfaktor von 1,3 wurde für die Berechnung benutzt:

| m/g | x/mm |
|---|---|
| 15 | 10 |
| 20 | 12 |
| 25 | 13 |
| 30 | 14 |
| 35 | 15 |
| 40 | 17 |

Darüber hinaus gibt es einige kritische Maße, die vor allem die Durchmesser der Schläuche und Rohre betreffen:

Die Innendurchmesser der Wasserleitung 31 und Ausgleichsleitung 33 sollten nicht kleiner als 2,3mm sein, da sonst Kapillarspannungen die Bewegung der Wassersäulen einschränken. Vorteilhaft ist, wenn die Wasserleitung 31 einen Innendurchmesser größer 4mm, ideal 6mm, aufweist, da dann eventuell vorhandene Luftblasen gut aufsteigen können und den Wasserfluss nicht behindern.

Aus demselben Grund sollte das Ausgleichsrohr 11 ca. 6mm Innendurchmesser haben, jedoch nicht weniger als 4mm.

Für den Fall der Ausführung ohne Ausgleichsrohr 11 sollte der Wasserauslass 14 mindestens 15mm Innendurchmesser aufweisen, optimal ca. 20mm.

Die Zwischenkammer 10 und der Schwimmer 20 können als Wegwerfteile (Engl.: disposables) konzipiert sein, so dass nur das Gehäuse 26 und der Deckel 5 gereinigt oder desinfiziert werden müssen.

Im Unterschied zu dem aus der WO2006/012887 A1 (anwaltliches Aktenzeichen SE31 P) bekannten Verdunster sind die in diesem Dokument beschriebenen Ausführungsformen für den Einsatz in Krankenhäusern gedacht. Dabei ist mit Luft oder Sauerstoff aus der zentralen Gasversorgung zu rechnen und der Einsatz kann 24 Stunden pro Tag erfolgen. Wegen des deutlich trockeneren Gases in dem Krankenhaus erhöht sich der spezifische Wasserverbrauch pro Stunde. Dem Klinikpersonal ist eine Befüllung öfter als einmal am Tag nicht zuzumuten. Damit steigt der benötigte Wasservorrat von etwa 250ml - 300ml bei Home Care auf 3I bei Clinical Care. Diese Wassermenge ist im Gerät nicht unterzubringen. Typisch ist die Anlieferung im 3I-Beutel, teilweise auch im Kanister/Tank.

Obwohl bisher von Wasser die Rede war, kann der erfindungsgemäße Atemgasbefeuchter auch für die Verdunstung anderer Flüssigkeiten wie beispielsweise von ätherischen Ölen verwendet werden. Obwohl bisher von Atemgas, was meist Luft ist, die Rede war, können auch beliebig andere Gase mit Flüssigkeitsmolekülen angereichert werden.

Der Wasseranschluss 18 und der Ausgleichsanschluss 19 können tatsächlich als trennbare Verbindungen ausgelegt sein. In einer anderen Ausführungsform können die Wasserleitung 31 und die Ausgleichsleitung 35 unlösbar mit der Zwischenkammer 10 verbunden sein, so dass der Wasseranschluss 18 und der Ausgleichsanschluss 19 nur den Übergangsbereich zwischen den Leitungen und der Zwischenkammer bezeichnen.

Anstelle des Nadelventils 17 kann auch ein anderes Ventil, beispielsweise mit planem Sitz, eingesetzt werden.

## Patentansprüche

1. Verdunstungskammer (2) mit:
einer Wanne (7); und
einer Zwischenkammer (10), die im Betrieb oberhalb der Wanne (7) -angeordnet ist,-mit einem Flüssigkeitsauslass (14) , der so angeordnet ist, dass im Betrieb durch den Flüssigkeitsauslass (14) Flüssigkeit (25) von der Zwischenkammer in die Wanne (7) fließen kann, wobei die Zwischenkammer (10) ein Pegelventil (17, 20) aufweist, das bestimmt und geeignet ist, im Betrieb aus einem Flüssigkeitsanschluss (18) Flüssigkeit in die Zwischenkammer nachlaufen zu lassen, so dass der Flüssigkeitspegel in der Zwischenkammer (10) zwischen einem Minimalpegel und einem Maximalpegel liegt,
**dadurch gekennzeichnet, dass**
der Boden der Zwischenkammer (10) den Flüssigkeitsauslass (14) aufweist.

2. Verdunstungskammer (2) gemäß Anspruch 1, **dadurch gekennzeichnet, dass** die Zwischenkammer (10) ferner einen Ausgleichsanschluss (19) aufweist, der pneumatisch mit dem Gasraum um den Schwimmer (20) herum pneumatisch verbunden ist.

3. Verdunstungskammer (2) gemäß einem der obigen Ansprüche, **dadurch gekennzeichnet, dass** das Pegelventil aufweist:
einen Schwimmer (20), der auf der Flüssigkeit (25) schwimmt, die in die Zwischenkammer (10) eingetreten ist; sowie
ein Ventil (17), das so mit dem Flüssigkeitsanschluss (18) verbunden ist, dass es den Flüssigkeitsanschluss (18) öffnen und verschließen kann, wobei das Ventil (17) so mit dem Schwimmer (20) mechanisch verbunden ist, dass der Schwimmer das Ventil schließt, wenn der Flüssigkeitspegel in der Zwischenkammer (10) den Maximalpegel überschritten hat und der Schwimmer das Ventil (17) öffnet, wenn der Flüssigkeitspegel in der Zwischenkammer (10) den Minimalpegel unterschritten hat.

4. Verdunstungskammer (2) gemäß Anspruch 3, wobei die Verdunstungskammer ferner unter der Zwischenkammer (10) eine Wanne (7) aufweist, wobei der Flüssigkeitsauslass (14) in dem Boden der Zwischenkammer so angeordnet ist, dass im Betrieb durch den Flüssigkeitsauslass (14) Flüssigkeit (25) von der Zwischenkammer in die Wanne (7) fließen kann.

5. Verdunstungskammer (2) gemäß einem der Ansprüche 1, 2 oder 4, **dadurch gekennzeichnet, dass** die Zwischenkammer (10) ferner ein Ausgleichsrohr (11) aufweist, dessen unteres Ende (13) im Betrieb unterhalb des Bodens der Zwischenkammer (10) und oberhalb des unteren Randes des Flüssigkeitsauslasses (14) liegt, wobei das obere Ende (12) des Ausgleichsrohrs (11) im Betrieb oberhalb des oberen Randes des Flüssigkeitsauslasses (14) liegt.

6. Verdunstungskammer (2) gemäß Anspruch 5, **dadurch gekennzeichnet, dass** sich das obere Ende (12) des Ausgleichsrohrs (11) oberhalb des Schwimmers (20) befindet, wobei das Ausgleichsrohr (11) im Betrieb vertikal angeordnet ist, wobei der Schwimmer (20) eine Aussparung (21) aufweist, durch die das Ausgleichsrohr (11) verläuft.

7. Verdunstungskammer (2) gemäß einem der Ansprüche 3 bis 6, soweit sich diese Ansprüche auf den Anspruch 4 rückbeziehen, **dadurch gekennzeichnet, dass** die Oberseite (22) des Schwimmers (20) im Betrieb von der Schwimmermitte unterhalb des Ventils (17) nach außen hin abfällt.

8. Verfahren zum Verdunsten einer Flüssigkeit (8) in einer Verdunstenkammer (2) nach einem der Ansprüche 1-7 mit:
Nachlaufen lassen von Flüssigkeit aus der Zwischenkammer (10), die oberhalb der Wanne (7) angeordnet ist, in die Wanne (7), so dass verdunstete Flüssigkeit ersetzt wird und
Nachlaufen lassen und von Flüssigkeit (34) aus dem Flüssigkeitsbehälter (33) durch das Pegelventil (17, 20) in die Zwischenkammer (10), wobei das Pegelventil (17, 20) das Nachlaufen der Flüssigkeit (34) so steuert, dass der Flüssigkeitspegel in der Zwischenkammer (10) zwischen einem Minimalpegel und einem Maximalpegel liegt;
**gekennzeichnet durch**:
Ausbilden des Flüssigkeitsfilms (8) in der Wanne (7); und
Nachlaufen lassen von Flüssigkeit aus der Zwischenkammer (10) in die Wanne (7) **durch** den Flüssigkeitsauslass (14) im Boden der Zwischenkammer (10), wobei die Dicke des Flüssigkeitsfilms (8) in etwa konstant bleibt.

9. Verfahren gemäß Anspruch 8, **dadurch gekennzeichnet, dass** eine Ausgleichsleitung (35) einen Druckunterschied zwischen dem Gasraum oberhalb der Flüssigkeit (34) im Flüssigkeitsbehälter (33) und dem Gasraum oberhalb der Flüssigkeit (25) in der Zwischenkammer (10) ausgleicht.

10. Verfahren gemäß einem der Ansprüche 8 und 9, **dadurch gekennzeichnet, dass** das Pegelventil aufweist:
einen Schwimmer (20), der auf der Flüssigkeit (25) schwimmt, die der Zwischenkammer (10) zugeführt wurde; sowie
ein Ventil (17), das so mit dem Flüssigkeitsbehälter (33) verbunden ist, dass es das Zuführen von Flüssigkeit (34) aus dem Flüssigkeitsbehälter (33) in die Zwischenkammer (10) steuern kann, wobei das Ventil (17) so mit dem Schwimmer (20) mechanisch verbunden ist, dass der Schwimmer das Ventil schließt, wenn der Flüssigkeitspegel in der Zwischenkammer (10) den Maximalpegel überschritten hat und der Schwimmer das Ventil (17) öffnet, wenn der Flüssigkeitspegel in der Zwischenkammer (10) den Minimalpegel unterschritten hat.

11. Verfahren gemäß einem der Ansprüche 8 bis 10, **dadurch gekennzeichnet, dass** das untere Ende (13) eines Ausgleichsrohrs (11) die Dicke des Flüssigkeitsfilms (8) in der Wanne (7) festlegt, wobei das untere Ende (13) des Ausgleichsrohrs (11) im Betrieb unterhalb des Bodens der Zwischenkammer (10) und oberhalb des unteren Randes des Flüssigkeitsauslasses (14) liegt, wobei das obere Ende (12) des Ausgleichsrohrs (11) im Betrieb oberhalb des oberen Randes des Flüssigkeitsauslasses (14) liegt.

12. Verfahren gemäß Anspruch 11, soweit sich der Anspruch 11 auf Anspruch 10 rückbezieht, **dadurch gekennzeichnet, dass** der Schwimmer (20) durch das Ausgleichsrohr (11) geführt wird, wobei der Schwimmer (20) eine Aussparung (21) aufweist, durch die das Ausgleichsrohr (11) verläuft.

13. Verfahren gemäß einem der Ansprüche 10 bis 12, soweit sich der Anspruch 11 auf den Anspruch 10 rückbezieht **dadurch gekennzeichnet, dass** aus dem Ventil (17) austretende Flüssigkeit auf der Oberseite (22) des Schwimmers (20) nach außen fließt.

## Claims

1. Evaporation chamber (2), comprising:
a trough (7); and
an intermediate chamber (10) which is disposed, in use, above the trough (7), having a liquid outlet (14) which is arranged to allow liquid (25) to flow, in use, from the intermediate chamber through the liquid outlet (14) into the trough (7), wherein the intermediate chamber (10) comprises a level valve (17, 20) which is intended and suited, in use, to allow a flow of liquid to follow from a liquid connection (18) into the intermediate chamber so that the liquid level in the intermediate chamber (10) is between a minimum level and a maximum level,
**characterized in that**
the bottom of the intermediate chamber (10) includes the liquid outlet (14).

2. Evaporation chamber (2) according to claim 1, **characterized in that** the intermediate chamber (10) further comprises a compensation connection (19), which is pneumatically connected to the gas space around the float (20).

3. Evaporation chamber (2) according to one of the preceding claims, **characterized in that** the level valve comprises:
a float (20) floating on the liquid (25) which has flown into the intermediate chamber (10); and
a valve (17) connected to the liquid connection (18) as to be able to open and close the liquid connection (18), wherein the valve (17) is mechanically connected to the float (20) such that the float closes the valve when the liquid level in the intermediate chamber (10) has exceeded the maximum level, and the float opens the valve (17) when the liquid level in the intermediate chamber (10) is lower than the minimum level.

4. Evaporation chamber (2) according to claim 3, wherein the evaporation chamber further includes a trough (7) underneath the intermediate chamber (10), wherein the liquid outlet (14) in the bottom of the intermediate chamber is arranged to allow liquid (25) to flow, in use, from the intermediate chamber through the liquid outlet (14) into the trough (7).

5. Evaporation chamber (2) according to one of claims 1, 2 or 4, **characterized in that** the intermediate chamber (10) further comprises a compensating pipe (11) whose lower end (13) is situated, in use, underneath the bottom of the intermediate chamber (10) and above the lower edge of the liquid outlet (14), wherein the upper end (12) of the compensating pipe (11) is situated, in use, above the upper edge of the liquid outlet (14).

6. Evaporation chamber (2) according to claim 5, **characterized in that** the upper end (12) of the compensating pipe (11) is located above the float (20), wherein the compensating pipe (11) is arranged vertically in use, wherein the float (20) comprises a recess (21) through which the compensating pipe (11) runs.

7. Evaporation chamber (2) according to one of claims 3 to 6, in as far as these claims depend on claim 4, **characterized in that**, in use, the upper side (22) of the float (20) is inclined downwards from the center of the float underneath valve (17) in an outward direction.

8. Method for evaporating a liquid (8) in an evaporation chamber (2) according to one of claims 1 to 7, comprising:
allowing a flow of liquid to follow from the intermediate chamber (10), which is disposed above the trough (7), into the trough (7) so that evaporated liquid is renewed and
allowing a flow of liquid (34) to follow from the liquid container (33) through the level valve (17, 20) into the intermediate chamber (10), wherein the level valve (17, 20) controls the continued flow of liquid (34) such that the liquid level in the intermediate chamber (10) is between a minimum level and a maximum level;
**characterized by**
forming a liquid film (8) in the trough (7); and
allowing a flow of liquid to follow from the intermediate chamber (10) into the trough (7) through the liquid outlet (14) in the bottom of the intermediate chamber (10), wherein the thickness of the liquid film (8) remains approximately constant.

9. Method according to claim 8, **characterized in that** a compensating conduit (35) compensates for a pressure difference between the gas space above the liquid (34) in the liquid container (33) and the gas space above the liquid (25) in the intermediate chamber (10).

10. Method according to one of claims 8 and 9, **characterized in that** the level valve comprises:
a float (20) floating on the liquid (25) supplied to the intermediate chamber (10); and
a valve (17) which is connected to the liquid container (33) as to be able to control the supply of liquid (34) from the liquid container (33) into the intermediate chamber (10), wherein the valve (17) is mechanically connected to the float (20) such that the float closes the valve when the liquid level in the intermediate chamber (10) has exceeded the maximum level, and the float opens the valve (17) when the liquid level in the intermediate chamber (10) is lower than the minimum level.

11. Method according to one of claims 8 to 10, **characterized in that** the lower end (13) of a compensating pipe (11) defines the thickness of the liquid film (8) in the trough (7), wherein the lower end (13) of the compensating pipe (11) is situated, in use, underneath the bottom of the intermediate chamber (10) and above the lower edge of the liquid outlet (14), wherein the upper end (12) of the compensating pipe (11) is situated, in use, above the upper edge of the liquid outlet (14).

12. Method according to claim 11, in as far as claim 11 depends on claim 10, **characterized in that** the float (20) is guided by the compensating pipe (11), wherein the float (20) comprises a recess (21) through which the compensating pipe (11) runs.

13. Method according to one of claims 10 to 12, in as far as claim 11 depends on claim 10, **characterized in that** liquid flowing out of the valve (17) flows to the outside on the upper side (22) of the float (20).

## Revendications

1. Chambre d'évaporation (2) avec:
un bac (7); et
une chambre intermédiaire (10) disposée en fonctionnement au-dessus du bac (7), avec une sortie de fluide (14) disposée de telle sorte qu'en fonctionnement, le fluide (25) peut s'écouler de la chambre intermédiaire jusque dans le bac (7) en passant par la sortie de fluide (14), la chambre intermédiaire (10) comportant une soupape à niveau (17, 20) définie et adaptée pour laisser sortir le fluide en fonctionnement du raccord de fluide (18) jusque dans la chambre intermédiaire, de sorte que le niveau de fluide se trouvant dans la chambre intermédiaire (10) se situe entre un niveau minimal et un niveau maximal;
**caractérisée en ce que**:
le fond de la chambre intermédiaire (10) comporte la sortie de fluide (14).

2. Chambre d'évaporation (2) selon la revendication 1, **caractérisée en ce que** la chambre intermédiaire (10) comporte en outre un raccord d'équilibrage (19) relié sur le plan pneumatique à l'espace gazeux entourant le flotteur (20).

3. Chambre d'évaporation (2) selon l'une quelconque des revendications précédentes, **caractérisée en ce que** la soupape à niveau comporte :
un flotteur (20) flottant sur le fluide (25) ayant pénétré la chambre intermédiaire (10); ainsi que
une soupape (17) reliée de telle sorte au raccord de fluide (18) qu'elle peut ouvrir et fermer le raccord de fluide (18), la soupape (17) étant reliée de telle sorte au flotteur (20), sur le plan mécanique, que le flotteur ferme la soupape lorsque le niveau de fluide se trouvant dans la chambre intermédiaire (10) a dépassé le niveau maximal et que le flotteur ouvre la soupape (17) lorsque le niveau de fluide se trouvant dans la chambre intermédiaire (10) est passé en dessous du niveau minimal.

4. Chambre d'évaporation (2) selon la revendication 3, la chambre d'évaporation comportant en outre un bac (7) placé sous la chambre intermédiaire (10), la sortie de fluide (14) étant disposée de telle sorte dans le fond de la chambre intermédiaire qu'en fonctionnement, le fluide (25) peut s'écouler de la chambre intermédiaire dans le bac (7) en passant par la sortie de fluide (14).

5. Chambre d'évaporation (2) selon l'une quelconque des revendications 1, 2 ou 4, **caractérisée en ce que** la chambre intermédiaire (10) comporte en outre un tube d'équilibrage (11) dont l'extrémité inférieure (13) se situe en fonctionnement en dessous du fond de la chambre intermédiaire (10) et au-dessus du bord inférieur de la sortie de fluide (14), l'extrémité supérieure (12) du tube d'équilibrage (11) se plaçant en fonctionnement au-dessus du bord supérieur de la sortie de fluide (14).

6. Chambre d'évaporation (2) selon la revendication 5, **caractérisée en ce que** l'extrémité supérieure (12) du tube d'équilibrage (11) se situe au-dessus du flotteur (20), le tube d'équilibrage (11) étant disposé, en fonctionnement, à la verticale, le flotteur (20) comportant un évidement (21) à travers lequel le tube d'équilibrage (11) passe.

7. Chambre d'évaporation (2) selon l'une quelconque des revendications 3 à 6, dans la mesure où ces revendications se réfèrent à la revendication 4, **caractérisée en ce que** le côté supérieur (22) du flotteur (20) retombe en fonctionnement vers l'extérieur, en dessous de la soupape (17), en partant du milieu du flotteur.

8. Procédé d'évaporation d'un fluide (8) dans une chambre d'évaporation (2) selon l'une quelconque des revendications 1 à 7 avec:
écoulement de fluide de la chambre intermédiaire (10) disposée au-dessus du bac (7) jusque dans le bac (7), de façon à remplacer le fluide évaporé; et écoulement de fluide (34) du réservoir de fluide (33) jusque dans la chambre intermédiaire (10) en passant par la soupape à niveau (17, 20), la soupape à niveau (17, 20) commandant de telle sorte l'écoulement de fluide (34) que le niveau de fluide se trouvant dans la chambre intermédiaire (10) se situe entre un niveau minimal et un niveau maximal;
**caractérisé par**:
la formation du film de fluide (8) dans le bac (7); et
l'écoulement de fluide de la chambre intermédiaire (10) jusque dans le bac (7) au travers de la sortie de fluide (14) située dans le fond de la chambre intermédiaire (10), de façon à ce que l'épaisseur du film de fluide (8) reste approximativement constante.

9. Procédé selon la revendication 8, **caractérisé en ce qu'**une conduite d'équilibrage (35) équilibre une différence de pression entre l'espace gazeux situé au-dessus du fluide (34) se trouvant dans le réservoir de fluide (33) et l'espace gazeux situé au-dessus du fluide (25) se trouvant dans la chambre intermédiaire (10).

10. Procédé selon l'une quelconque des revendications 8 et 9, **caractérisé en ce que** la soupape à niveau présente:
un flotteur (20) flottant sur le fluide (25) amené dans la chambre intermédiaire (10) ; ainsi que
une soupape (17) reliée de telle sorte au réservoir de fluide (33) qu'elle peut commander l'amenée de fluide (34) du réservoir de fluide (33) jusque dans la chambre intermédiaire (10), la soupape (17) étant reliée de telle sorte sur le plan mécanique au flotteur (20) que le flotteur ferme la soupape lorsque le niveau de fluide se trouvant dans la chambre intermédiaire (10) a dépassé le niveau maximal et que le flotteur ouvre la soupape (17) lorsque le niveau de fluide se trouvant dans la chambre intermédiaire (10) se situe en dessous du niveau minimal.

11. Procédé selon l'une quelconque des revendications 8 à 10, **caractérisé en ce que** l'extrémité inférieure (13) d'un tube d'équilibrage (11) détermine l'épaisseur du film de fluide (8) dans le bac (7), l'extrémité inférieure (13) du tube d'équilibrage (11) se situant, en fonctionnement, en dessous du fond de la chambre intermédiaire (10) et au-dessus du bord inférieur de la sortie de fluide (14), l'extrémité supérieure (12) du tube d'équilibrage (11) se situant, en fonctionnement, au-dessus du bord supérieur de la sortie de fluide (14).

12. Procédé selon la revendication 11, dans la mesure où la revendication 11 se réfère à la revendication 10, **caractérisé en ce que** le flotteur (20) est guidé par le tube d'équilibrage (11), le flotteur (20) comportant un évidement (21) à travers lequel le tube d'équilibrage (11) s'étend.

13. Procédé selon l'une quelconque des revendications 10 à 12, dans la mesure où la revendication 11 se réfère à la revendication 10, **caractérisé en ce que** le fluide sortant de la soupape (17) s'écoule vers l'extérieur, sur le côté supérieur (22) du flotteur (20).
